# EUROPEAN PATENT APPLICATION

(11) **EP 4 424 708 A1**
(43) Date of publication of application: **04.09.2024**
(21) Application number: 22887514.2
(22) Date of filing: 24.10.2022
(51) Int. Cl.: C07K 16/28, A61P 35/00, A61K 39/00

(54) **ANTIBODY DERIVED FROM CANCER SURVIVOR, AND USE THEREOF**

(30) Priority: 26.10.2021 KR 20210143692
(71) Applicant: The Asan Foundation, Seoul 05505 (KR); University Of Ulsan Foundation For Industry Cooperation, Ulsan 44610 (KR)
(72) Inventor: CHANG, Su Hwan, Namyangju-si Gyeonggi-do 12225 (KR); LEE, Jong Won, Seoul 06627 (KR); LEE, Sae Byul, Seoul 05505 (KR); CHOI, Eun Young, Seoul 05505 (KR); KIM, Yong Sub, Seoul 05505 (KR); KIM, Geon Yeong, Daejeon 34141 (KR); SON, Su Min, Daejeon 34141 (KR)
(74) Representative: AWA Sweden AB
(86) International application number: PCT/KR2022/016212
(87) International publication number: WO 2023/075318

(57) **Abstract**

The present disclosure relates to an antibody derived from a cancer survivor and use thereof. More particularly, among antibodies derived from survivors of 5 or more years from breast cancer surgery, an antibody showing high binding strength to cancer cells and having an inhibitory effect on the growth and migration of cancer cells is selected to identify an antigen of the antibody. The antibody is then highly likely to contribute to the survival of cancer patients and unlikely to cause adverse effects upon administration, and thus, can be widely used clinically for preventing and treating cancer and inhibiting metastasis.

## Description

### [Technical field]

The present disclosure relates to an antibody derived from a cancer survivor and use thereof, and more particularly, to a cancer cell-specific antibody derived from the blood of a survivor of 5 or more years after breast cancer surgery and use of the cancer cell-specific antibody for treating or preventing cancer.

### [Background art]

Cancer, which is one of the leading causes of death in modern people, is a disease caused by changes in normal cells due to various causes, and refers to a tumor that does not follow differentiation, proliferation, growth patterns, etc. of normal cells and is malignant. Cancer is characterized by "uncontrolled cell growth," and due to such abnormal cell growth, a mass of cells, called a tumor, is formed. Tumors infiltrate surrounding tissues, and in severe cases, may metastasize to other organs of the body.

Cancer is an incurable, chronic disease that, despite treatment with surgery, radiation, and drug therapy, cannot be fundamentally cured in many cases, causing suffering and ultimately death. In particular, the global cancer incidence rate has recently been increasing by more than 5 % every year due to the increasing number of elderly people, environmental degradation, etc.

Meanwhile, drug treatment, i.e., use of anticancer drugs, for cancer typically treats cancer by attacking and killing cancer cells with compounds that are generally cytotoxic. However, drug treatment damages not only cancer cells but also normal cells, resulting in high side effects. Therefore, targeted anticancer drugs have been developed to reduce side effects. However, while such targeted anticancer drugs have been able to reduce side effects, they have been limited by a high rate of resistance.

Therefore, there has been a recent surge of interest in immuno-oncology drugs that utilize the immune system of the body to reduce problems caused by toxicity and resistance. As an example of such immune-oncology drugs, immune checkpoint inhibitors, which specifically bind to PD-1 on the surface of cancer cells and inhibit binding of T cells to PD-1 to activate T cells to attack cancer cells, have been developed. However, even such immune checkpoint inhibitors have limitations in that they are not effective in a wide range of cancers.

However, to date, no studies have been reported on antibodies derived from cancer survivors showing anti-cancer effects. Antibodies derived from existing cancer patients are unlikely to have anticancer effects, but in people who have survived cancer surgery for a long period of time, antibodies that contributed to the survival are likely to be present.

Accordingly, the present inventors selected antibodies showing high binding strength to cancer cells among antibodies isolated from the blood of survivors of 5 or more years after cancer surgery, confirmed the effects of the selected antibodies in inhibiting growth and migration of cancer cells, and identified antigens of the selected antibodies, thereby completing the present disclosure.

### [Description of embodiments]

### [Technical problem]

An object of the present disclosure is to provide, as an antibody derived from a cancer survivor, an antibody and an antigen-binding fragment thereof that exhibits an inhibitory effect on growth and migration of cancer cells.

Another object is to provide a polynucleotide encoding the antibody or the antigen-binding fragment thereof.

Another object is to provide a recombinant vector including the polynucleotide.

Another object is to provide a host cell transformed by introducing the recombinant vector.

Another object is to provide a method of producing an antibody or an antigen-binding fragment thereof by culturing a transformed cell line.

Another object is to provide a pharmaceutical composition for treating or preventing cancer, including the antibody or the antigen-binding fragment.

Another object is to provide use of the antibody or the antigen-binding fragment for preventing or treating cancer.

Another object is to provide a method of preventing or treating cancer, the method including administering to a subject a composition including the antibody or the antigen-binding fragment as an active ingredient.

### [Solution to problem]

To achieve the objects above, one aspect provides an anti-ICAM1 antibody or an antigen-binding fragment thereof, including: a heavy chain variable region including a heavy chain CDR1 having an amino acid sequence of SEQ ID NO: 1 or 10, a heavy chain CDR2 having an amino acid sequence of SEQ ID NO: 2 or 11, and a heavy chain CDR3 having an amino acid sequence of SEQ ID NO: 3 or 12; and a light chain variable region including a light chain CDR1 having an amino acid sequence of SEQ ID NO: 5 or 14, a light chain CDR2 having an amino acid sequence of SEQ ID NO: 6 or 15, and a light chain CDR3 having an amino acid sequence of SEQ ID NO: 7 or 16.

The anti-ICAM1 antibody or the antigen-binding fragment thereof may include the heavy chain variable region and the light chain variable region in one of the following combinations: a) the heavy chain variable region including the heavy chain CDR1 having the amino acid sequence of SEQ ID NO: 1, the heavy chain CDR2 having the amino acid sequence of SEQ ID NO: 2, and the heavy chain CDR3 having the amino acid sequence of SEQ ID NO: 3; and the light chain variable region including the light chain CDR1 having the amino acid sequence of SEQ ID NO: 5, the light chain CDR2 having the amino acid sequence of SEQ ID NO: 6, and the light chain CDR3 having the amino acid sequence of SEQ ID NO: 7; and b) the heavy chain variable region including the heavy chain CDR1 having the amino acid sequence of SEQ ID NO: 10, the heavy chain CDR2 having the amino acid sequence of SEQ ID NO: 11, and the heavy chain CDR3 having the amino acid sequence of SEQ ID NO: 12; and the light chain variable region including the light chain CDR1 having the amino acid sequence of SEQ ID NO: 14, the light chain CDR2 having the amino acid sequence of SEQ ID NO: 15, and the light chain CDR3 having the amino acid sequence of SEQ ID NO: 16.

In the anti-ICAM1 antibody or the antigen-binding fragment thereof, the heavy chain variable region may include an amino acid sequence of SEQ ID NO: 4 or 13, and the light chain variable region may include an amino acid sequence of SEQ ID NO: 8 or 17.

The anti-ICAM1 antibody or the antigen-binding fragment thereof may include the heavy chain variable region and the light chain variable region in one of the following combinations: a) the heavy chain variable region including the amino acid sequence of SEQ ID NO: 4 and the light chain variable region including the amino acid sequence of SEQ ID NO: 8; and b) the heavy chain variable region including the amino acid sequence of SEQ ID NO: 13 and the light chain variable region including the amino acid sequence of SEQ ID NO: 17.

The anti-ICAM1 antibody may include an amino acid sequence of SEQ ID NO: 9 or 18. The anti-ICAM1 antibody may consist of an amino acid sequence of SEQ ID NO: 9 or 18. The anti-ICAM1 antibody may have an amino acid sequence with a sequence identity of at least 80 %, at least 85 %, at least 90 %, at least 95 %, at least 96 %, at least 97 %, at least 98 %, or at least 99 % to the amino acid sequence of SEQ ID NO: 9 or 18.

In an embodiment, S3-5 and S4-2 antibodies showing high binding strength to cancer cells were selected from the blood of patients who have survived 5 or more years after triple-negative breast cancer surgery, and antigens of the selected antibodies were identified. As a result, when the expression of ICAM 1 is reduced, the binding strength of the S3-5 and S4-2 antibodies to cancer cells was found to be weakened, indicating that ICAM1 serves an antigen. In addition, based on the presence of ICAM1 in the product of immunoprecipitation of the S3-5 and S4-2 antibodies, it can be verified that an antigen of the S3-5 and S4-2 antibodies is ICAM1.

The term "intercellular adhesion molecule 1 (ICAM1)" is also called CD54, and may refer to an inflammatory protein involved in the attachment and movement of inflammatory cells. The ICAM1 may be a type of intercellular adhesion molecule that is constantly present at low concentrations in the membranes of leukocytes and endothelial cells, and the concentration of the ICAM1 may increase significantly upon cytokine stimulation.

The term "antibody" refers to an immunoglobulin (Ig) molecule that is immunologically reactive with a specific antigen, and may refer to a protein molecule that acts as a receptor that specifically recognizes an antigen. This term may include both a whole antibody and an antibody fragment. The whole antibody generally consists of two pairs of a light chain (LC) and a heavy chain (HC), which are polypeptides consisting of several domains, or consists of a two-pair structure of HC/LC as a basic unit. There are five types of heavy chains that constitute mammalian antibodies, denoted by the Greek letters α, δ, ε, γ, and µ. Depending on the types of the heavy chain, different types of antibodies such as IgA, IgD, IgE, IgG, and IgM may be produced. There are two types of the light chains that constitute mammalian antibodies, denoted by λ and κ. The light and heavy chains are connected by one covalent disulfide bond, with the variable and constant regions of each of the chains aligned side by side, and the heavy chains of two molecules bound to the light chain are connected by two shared disulfide bonds to form the whole antibody. The whole antibody binds specifically to an antigen through the variable regions of the heavy and light chains. Since the whole antibody consists of two pairs of heavy and light chains (HC/LC), the whole antibody in one molecule may have divalent specificity that binds to the same two antigens through two variable regions. The variable region of an antibody that binds to an antigen is called an antigen-binding site of the antibody, and the part on the surface of the antigen recognized by the antibody may be called an epitope.

The term "heavy chain" can be meant to include both a full-length heavy chain and a fragment thereof, including a variable region (VH) and three constant regions, CH1, CH2, and CH3, sufficient to confer specificity for the antigen.

The term "light chain" can be meant to include both a full-length light chain and a fragment thereof, including a variable region (VL) and a constant region (CL) sufficient to confer specificity for the antigen.

The term "CDR" may refer to a complementarity determining region that is part of the VH and the VL of the antibody.

The variable region of the antibody including the antigen-binding site may be subdivided into a framework region (FR) with low sequence variability and a complementarity-determining region which is a hypervariable region with high sequence variability. The VH and VL each have three CDRs and four FRs, arranged from an N-terminus to a C-terminus in the following order: FR1-CDR1-FR2-CDR2-FR3-CDR3-FR4. Even within the variable regions of the antibody, a CDR with the highest sequence variability is a site of direct binding to the antigen, and thus may be the most important for the antigen specificity of the antibody.

The anti-ICAM1 antibody may include not only a whole antibody form that binds specifically to ICAM1, but also an antigen-binding fragment of the antibody molecule.

The anti-ICAM1 antibody is an antibody in which a specific amino acid sequence is included in CDRs of the light and heavy chains to enable ICAM1-specific binding, and may include both a monoclonal antibody and a polyclonal antibody. Particularly, the anti-ICAM1 antibody may be a monoclonal antibody.

In addition, the antibody may include all of a chimeric antibody, a humanized antibody, and a human antibody, and for example, may be a human antibody.

The fragments of the antibody may be in the form of Fv, single-chain Fvs (scFV), single-chain antibodies, Fab, F(ab'), F(ab')2, or disulfide-linked Fvs (di-scFV).

Fragment antigen-binding (Fab) is an antigen-binding fragment of an antibody, which may consist of one variable domain and one constant domain in each of the heavy chain and the light chain. F(ab')2 is a fragment produced by hydrolyzing an antibody with pepsin, and may consist of two Fabs connected by a disulfide bond at the heavy chain hinge. F(ab') may be a monomeric antibody fragment in the form of a heavy chain hinge attached to Fab separated by reduction of the disulfide bond of the F(ab')2 fragment. Variable fragment (Fv) may be an antibody fragment consisting of only the variable regions of each of the heavy chain and the light chain. Single-chain variable fragment (scFv) may be a recombinant antibody fragment in which the VH and the VL are connected by a flexible peptide linker. di-scFV may refer to a fragment in which a VH and a VL of scFv are connected by a very short linker and thus are unable to bind to each other, but are able to a VH and a VL of another scFv of the same conformation, respectively, to form a dimer.

The antibody or the antigen-binding fragment thereof may include conservative amino acid substitutions that do not substantially alter the biological activity of the antibody (referred to as conservative variants of the antibody).

Another aspect provides a polynucleotide encoding the antibody or the antigen-binding fragment thereof.

The polynucleotide may be modified. The modification includes addition, deletion, or non-conservative or conservative substitution of nucleotides.

A polynucleotide encoding the anti-ICAM1 antibody may include a nucleotide sequence of SEQ ID NO: 19 or 20. The polynucleotide encoding the anti-ICAM1 antibody may include a nucleotide sequence of SEQ ID NO: 19 or 20. The polynucleotide may have a nucleotide sequence with a sequence identity of at least 80 %, at least 85 %, at least 90 %, at least 95 %, at least 96 %, at least 97 %, at least 98 %, or at least 99 % to the nucleotide sequence of SEQ ID NO: 19 or 20.

Another aspect provides a recombinant vector including the polynucleotide.

The term "vector" may refer to a means for expressing a target gene in a host cell. For example, the vector may include a viral vector such as a plasmid vector, a cosmid vector, a bacteriophage vector, an adenovirus vector, a retrovirus vector, and an adeno-associated virus vector. Vectors that can be used as the recombinant vector may include plasmids often used in the art (e.g., pSC101, pGV1106, pACYC177, ColE1, pKT230, pME290, pBR322, pUC8/9, pUC6, pBD9, pHC79, pIJ61, pLAFR1, pHV14, pGEX series, pET series, pUC19, and p426GPD , etc.), phages (e.g., λgt4λB, λ-Charon, λ△z1 and M13, etc.), or viruses (e.g., CMV, SV40, etc.), but are not limited thereto. Since plasmids are currently the most commonly used form of vectors, a "plasmid" and a "vector" may be sometimes used interchangeably.

In the recombinant vector, the polynucleotide encoding the antibody or the antigen-binding fragment thereof may be operably linked to a promoter. The term "operably linked" refers to a functional connection between a promoter sequence, which initiates and mediates transcription of a polynucleotide encoding a target peptide, and a sequence of the polynucleotide.

The recombinant vector may be typically constructed as a vector for cloning or expression. For the expression vector, a conventional vector used in the art to express a foreign protein in plants, animals, or microorganisms may be used. The recombinant vector may be constructed through various methods known in the art.

The recombinant vector may be constructed by using prokaryotic cells or eukaryotic cells as a host. For example, when a vector in use is an expression vector and a prokaryotic cell is used as a host, the vector generally include a strong promoter capable of progressing transcription (e.g., a pLλ promoter, a trp promoter, a lac promoter, a tac promoter, a T7 promoter, etc.), a riboosme-binding site, and a sequence regulating the termination of transcription/translation. When a eukaryotic cell is used as a host, replication origins included in the vector may include an f1 replication origin, an SV40 replication origin, a pMB1 replication origin, an adeno replication origin, an AAV replication origin, a CMV replication origin, a BBV replication origin, etc., but embodiments are not limited thereto. In addition, a promoter derived from the genome of mammalian cells (e.g., metallotionine promoter) or a promoter derived from mammalian viruses (e.g., an adenovirus late promoter, a vaccinia virus 7.5K promoter, an SV40 promoter, a cytomegalovirus (CMV) promoter, and an HSV tk promoter) may be used, which generally has a polyadenylation sequence as a transcription termination sequence.

Another aspect provides a host cell transformed by introducing the recombinant vector.

The term "transformation" refers to a modification of the genotype of a host cell by introducing a foreign polynucleotide, and may refer to introduction of a foreign polynucleotide into a host cell regardless of methods used for the transformation.. A foreign polynucleotide introduced into a host cell may be integrated and maintained within the genome of the host cell, or may remain unintegrated.

For use as a host cell that can be transformed by introducing a recombinant vector, a host having high efficiency of DNA introduction and high expression efficiency of the introduced DNA may be generally used. For example, known eukaryotic and prokaryotic hosts of *Escherichia coli, Pseudomonas, Bacillus, Streptomyces,* fungi, and yeast; insect cells such as Spodoptera frugiperda (SF9) cells; and animal cells such as CHO, COS 1, COS 7, BSC 1, BSC40, BMT 10, etc., may be used, but are not limited thereto.

The introduction or insertion of a polynucleotide or a recombinant vector including the same into a host cell may be performed by a method widely known in the art. As a delivery method, for example, a calcium chloride (CaCl₂) method, an electroporation method, or the like may be used when a host cell is a prokaryotic cell, or a microinjection method, a calcium phosphate precipitation method, an electroporation method, a liposome-mediated transfection method, a gene bombardment method, or the like may be used when a host cell is a eukaryotic cell.

Another aspect provides a method of preparing the antibody or the antigen-binding fragment thereof, the method including: culturing the transformed host cell; and obtaining the antibody or the antigen-binding fragment thereof from the cultured cell line.

In the culturing, a medium and culture conditions may be appropriately selected and used from those generally utilized for host cells, and the culture conditions such as temperature, incubation time, and the like may be adjusted to suit mass production of the antibody or the antigen-binding fragment thereof. The antibody or the antigen-binding fragment thereof expressed following the culturing may be isolated from cell lysates. When transformants that have been overexpressed are centrifuged to obtain cell pellets, and medium components including ruptured cell walls or ruptured cell membranes are removed, an eluate containing cell lysates may be obtained. Here, the cell pellets may be lysed by methods well known in the art, such as use of alkalis, surfactants (e.g., CHAPS, SDS, etc.), organic solvents, and enzymes (e.g., lysozyme), sonication, and utilization of high-pressure grinder (e.g., French Press), and periodic application of high temperature, pressure, cycles of freezing and thawing, etc. In addition, the antibody or the antigen-binding fragment thereof may be purified and isolated pure.

Another aspect provides a pharmaceutical composition for treating or preventing cancer, including the antibody or the antigen-binding fragment thereof.

In one or more embodiments, it can be confirmed that selected S3-5 and S4-2 antibodies inhibit the growth and migration of cancer cells and promote the apoptosis of cancer cells by activating immune cells through *in vitro* experiments. In addition, through *in vivo* experiments, it can be confirmed that administration of the S3-5 and S4-2 antibodies reduces the amount of cancer cells that have metastasized in a mouse model of breast cancer with lung metastasis. Therefore, it can be confirmed that the S3-5 and S4-2 antibodies that bind specifically to ICAM1 have effects of treating and preventing cancer and inhibiting metastasis.

The term "cancer" refers to a group of diseases caused by cells that have aggressive characteristics in which cells divide and proliferate without respect to normal growth limits, invasive characteristics in which cells invade the surrounding tissue, and metastatic characteristics in which cells spread to other parts of the body.

The cancer may be breast cancer, stomach cancer, liver cancer, lung cancer, colon cancer, colorectal cancer, prostate cancer, ovarian cancer, pancreatic cancer, cervical cancer, thyroid cancer, laryngeal cancer, renal cancer, skin cancer, acute myeloid leukemia, brain tumor, neuroblastoma, retinoblastoma, head and neck cancer, salivary gland cancer, or lymphoma, but is not limited thereto. In detail, the cancer may be breast cancer. In more detail, the cancer may be triple negative breast cancer.

The pharmaceutical composition may include, as an active ingredient, the antibody or the antigen-binding fragment thereof in an amount of about 0.1 wt% to about 90 wt%, specifically, about 0.5 wt% to about 75 wt%, more specifically, about 1 wt% to about 50 wt%, based on the total weight of the composition.

The pharmaceutical composition may include conventional non-toxic pharmaceutically acceptable additives that are mixed into preparations according to conventional methods. For example, the pharmaceutical composition may further include a pharmaceutically acceptable carrier, diluent, or excipient.

Examples of additives used in the pharmaceutical composition may include sweeteners, binders, solvents, dissolution aids, wetting agents, emulsifiers, isotonic agents, absorbents, disintegrants, antioxidants, preservatives, lubricants, glidants, fillers, flavoring agents, etc. For example, the additives may include lactose, dextrose, sucrose, mannitol, sorbitol, cellulose, glycine, silica, talc, stearic acid, stearin, magnesium stearate, magnesium aluminosilicate, starch, gelatin, gum tragacanth, alginic acid, sodium alginate, methylcellulose, sodium carboxymethylcellulose, agar, water, ethanol, polyethylene glycol, polyvinylpyrrolidone, sodium chloride, calcium chloride, orange essence, strawberry essence, vanilla flavor, etc.

The pharmaceutical composition may be formulated in various formulation forms for oral administration (e.g., tablets, pills, powders, capsules, syrups, or emulsions) or parenteral administration (e.g., intramuscular, intravenous, or subcutaneous injection).

In detail, the pharmaceutical composition may be formulated into a formulation for oral administration, and additives used hereinin may include cellulose, calcium silicate, corn starch, lactose, sucrose, dextrose, calcium phosphate, stearic acid, magnesium stearate, calcium stearate, gelatin, talc, surfactants, suspending agents, emulsifiers, diluents, etc. In addition, examples of lubricants may include colloidal silicon dioxide, magnesium silicate, etc.; examples of diluents may include microcrystalline cellulose, lactose anhydrous, lactose monohydrate, and silicified MCC HD 90, etc.; and examples of disintegrants may include croscarmellose sodium, crospovidone, etc.; and examples of lubricants may include magnesium stearate, sodium lauryl sulfate, stearic acid, etc.

In addition, examples of liquid formulations for oral administration may include suspensions, emulsions, syrups, etc., and may include various excipients, such as wetting agents, sweetening agents, fragrances, preservatives, etc., in addition to simply diluents that are commonly used, such as water and liquid paraffin.

In addition, formulations for parenteral administration may include sterile aqueous solutions, non-aqueous solvents, suspensions, emulsions, freeze-dried agents, and suppositories. Examples of non-aqueous solvents and suspensions may include propyleneglycol, polyethylene glycol, plant oil, such as olive oil, and injectable ester such as ethyloleate. As a base for suppositories, witepsol, macrogol, tween 61, cacao butter, laurinum, glycerogelatin, etc. may be used. Meanwhile, injectables may contain conventional additives such as solubilizers, isotonic agents, suspending agents, emulsifiers, stabilizers, preservatives, etc.

The pharmaceutical composition may be administered to a patient in a therapeutically effective amount or a pharmaceutically effective amount.

The term "therapeutically effective amount" or "pharmaceutically effective amount" is an amount of a compound or composition effective in preventing or treating a target disease, and refers to an amount that is sufficient to treat a disease with a reasonable benefit/risk ratio applicable to medical treatment and that does not cause side effects. The level of the effective amount may be determined by factors including health conditions of a patient, type and severity of a disease, activity of drugs, sensitivity to drugs, methods of administration, time of administration, route of administration, rate of release, duration of treatment, and drugs combined or used simultaneously, and by other factors well known in the medical field.

The pharmaceutical composition may be administered as an individual therapeutic agent, may be administered in combination with other therapeutic agents, may be administered sequentially or simultaneously with conventional therapeutic agents, and may be administered once or multiple times. Considering all of the factors above, it is important to administer an amount that can achieve maximum effect with minimum amount without side effects, and such an amount may be easily determined by those skilled in the art.

In detail, the effective amount of the antibody or the antigen-binding fragment thereof in the pharmaceutical composition may vary depending on the age, gender, and weight of a patient, and may typically be, per kilogram of body weight, from about 0.1 mg to about 1,000 mg or about 5 mg to about 5 mg, administered daily or every other day, or in one to three divided daily doses. However, since the effective amount may increase or decrease depending on the route of administration, severity of disease, gender, weight, age, etc., the range is not limited thereto.

In addition, the pharmaceutical composition may be administered for tumor therapy in combination with chemotherapy, radiation therapy, immunotherapy, hormone therapy, bone marrow transplantation, stem cell replacement therapy, other biological treatments, surgical intervention, or combinations thereof. For example, the pharmaceutical composition may be used for adjuvant therapy in combination with other long-term treatment strategies, or may be used to regress tumors in critically ill patients or to maintain the condition of a patient after chemotherapy.

Another aspect provides use of the antibody or the antigen-binding fragment for preventing or treating cancer.

Another aspect provides a method of preventing or treating cancer, the method including administering to a subject the composition including the antibody or the antigen-binding fragment as an active ingredient.

Redundant contents are omitted in consideration of the complexity of the present specification, and terms not otherwise defined in the present specification have meanings commonly used in the technical field to which the present invention pertains.

### [Advantageous effects of disclosure]

The antibody according to one aspect shows high binding strength to cancer cells among antibodies derived from the blood of a person who has survived breast cancer surgery for more than 5 years, and the antigen of the antibody is identified by selecting antibodies that exhibit inhibitory effects on the growth and migration of cancer cells. In this regard, the antibody disclosed herein is likely to be an antibody that contributes to the survival of a cancer patient, and is unlikely to cause side effects upon administration, and thus may be widely used clinically for the prevention and treatment of cancer and inhibition of metastasis of cancer.

### [Brief description of drawings]

FIG. 1 shows the results of flow cytometry performed to select antibodies showing high binding strength to cancer cells among antibodies derived from the blood of a person who has survived 5 or more years after triple-negative breast cancer surgery according to one aspect:

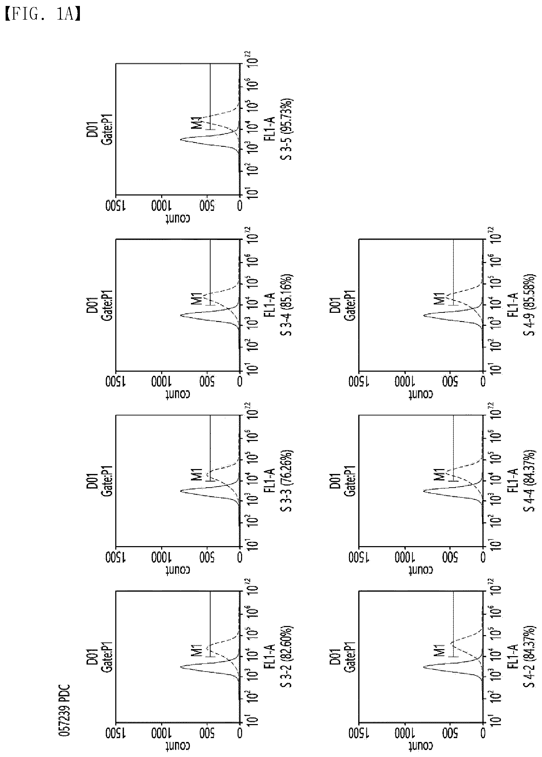

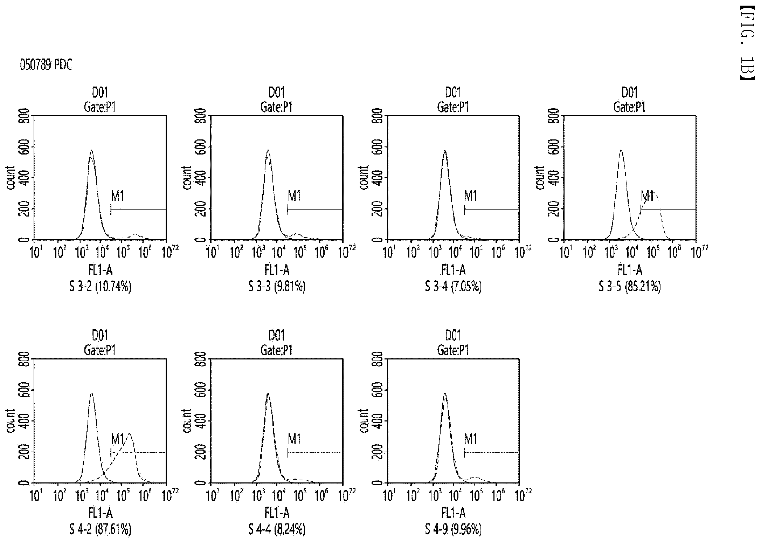

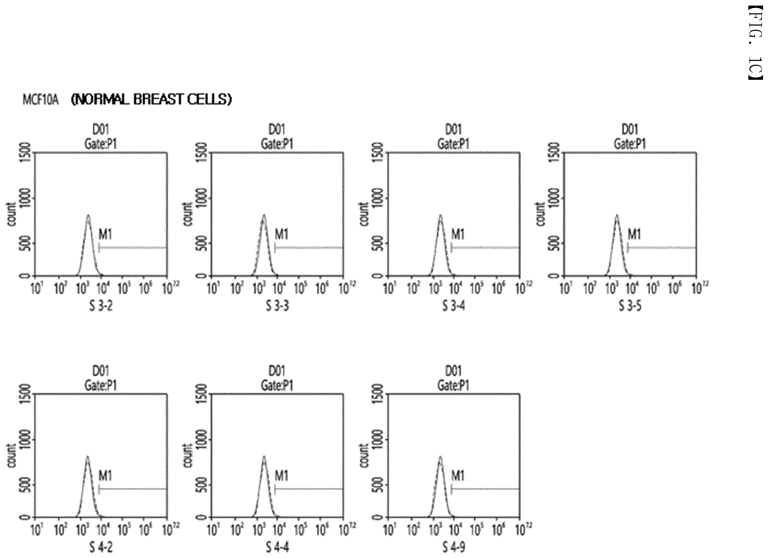

FIG. 1A shows graphs of the binding strength between antibodies derived from the blood of a survivor of 5 or more years after triple-negative breast cancer surgery and cancer cells obtained from a survivor of 5 or more years after triple-negative breast cancer surgery; FIG. 1B shows graphs of the binding strength between antibodies derived from the blood of a person who has survived 5 or more years after triple-negative breast cancer surgery and cancer cells obtained from a person who has survived 5 or more years after triple-negative breast cancer surgery; and FIG. 1C shows graphs of the binding strength between antibodies derived from the blood of a survivor of 5 or more years after triple-negative breast cancer surgery and an epithelial cell line from breast tissue.
FIG. 2 shows the results of *in vitro* cell proliferation analysis to determine effects of antibodies selected according to one aspect on the growth of cancer cells.
FIG. 3 shows images obtained by using an optical microscope after performing *in vitro* cell migration analysis to determine effects of antibodies selected according to one aspect on the migration of cancer cells.
FIG. 4 shows the results of measuring the ratio of migrated cells after performing *in vitro* cell migration analysis to determine effects of antibodies selected according to one aspect on the migration of cancer cells.
FIG. 5 shows photographs of lung tissue with stained cancer cells after administration of a selected antibody to an animal model of breast cancer with lung metastasis, to determine effects of the administration of antibodies selected according to one aspect on the animal model of breast cancer with lung metastasis.
FIG. 6 shows the results of measuring a metastatic index, which quantifies the area of lung where cancer cells have metastasized after administration of a selected antibody to an animal model of breast cancer with lung metastasis, to determine effects of the administration of antibodies selected according to one aspect on the animal model of breast cancer with lung metastasis.
FIG. 7 shows the results of antibody-dependent cell cytotoxicity (ADCC) assay performed by using antibodies selected according to one aspect.
FIG. 8 shows the results of determining whether there is a change in antibody binding by FACS after ICAM1 gene knockdown by using siRNA, to identify antigens of the antibodies selected according to one aspect.
FIG. 8A shows graphs showing the binding ability of breast cancer cell line MDA-MB-468, S3-5, S4-2, and Cetuximab; FIG. 8B shows graphs showing the binding ability of breast cancer cell line MDA-MB-468, S3-5, and S4-2, each treated with ICAM1 siRNA, and the binding ability of breast cancer cell line MDA-MB-468 and Cetuximab, each treated with siEGFR; and FIG. 8C shows graphs showing the binding ability of breast cancer cell line MDA-MB-468, S3-5, and S4-2, each treated with ICAM1 siRNA, and the binding ability of breast cancer cell line MDA-MB-468 and Cetuximab, each treated with siEGFR.
FIG. 9 shows the results of western blot performed to determine whether ICAM1 is present in a reaction product of immunoprecipitation of S3-5 antibody selected by one aspect.
FIG. shows the results of western blot performed to determine whether ICAM1 is present in a reaction product of immunoprecipitation of S4-2 antibody selected by one aspect.

### [Best model

Hereinafter, the present disclosure will be described in detail in Examples below. However, these Examples are for illustrative purposes only, and the scope of the present disclosure is not intended to be limited by these Examples.

### Example 1. Construction of antibody library from breast cancer survivors

An antibody library from survivors of 5 or more years after triple-negative breast cancer surgery was constructed as follows. cDNA was synthesized from RNA extracted from lymph node (LN) and peripheral blood mononuclear cell (PBMC) samples of survivors of 5 or more years after triple-negative breast cancer surgery, and genes of λ chain (Vλ) and κ chain (Vκ) of the heavy chain variable region (VH) and the light chain variable region (LH) were amplified by a polymerase chain reaction (PCR). Assembly PCR was performed to combine the heavy chain and the light chain into the scFv form, and the combined scFv genes were amplified by extension PCR.

Next, to construct an scFv library, the amplified scFv genes was inserted into a phagemid vector, pDR-D1. The pDR-D1 vector with the amplified scFv genes was digested with Sfil restriction enzyme, and then library cloning was performed thereon through ligation. The scFv library plasmid thus constructed was electroporated into *E*. *coli* ER2738 for transformation, thereby obtaining an antibody library with a final size of about 2×10⁸ cfu.

### Example 2. Identification of antibodies binding specifically to cancer cells

To identify antibodies binding specifically to cancer cells among the antibody library prepared in Example 1, biopanning was performed as follows. For biopanning, MDA-MB-468 cells (ATCC, USA; HTB-132^{™}) which are the breast cancer cell line were used, and MCF-10A cells (ATCC, USA; CRL-10317^{™}) which are epithelial cell line of breast tissue were used as a negative control cell line. A total of four rounds of biopanning were performed, and in each panning round, library phages were first treated with MCF-10A cells which are a negative control cell line to maximize the removal of phages binding to the MCF-10A cells. Then, only the supernatant containing phages not binding to the MCF-10A cells was isolated and treated with MDA-MB-468 which is a target cell. Afterwards, phages not binding to the MDA-MB-468 cells were removed by washing, and only those that were still bound to the MDA-MB-468 cells were eluted and re-infected into *E0*. *coli.* This procedure was repeatedly performed to amplify the phages binding to the MDA-MB-468 cells. By sequencing of the amplified phages after the third panning and the fourth panning, seven antibodies (S3-2, S3-3, S3-4, S3-5, S4-2, S4-4, S4-9) binding specifically to the MDA-MB-468 cells were identified.

Afterwards, to produce and purify experimental antibodies, the variable regions of each the seven identified antibodies were cloned into a whole IgG expression vector, and then transfected into HEK293E cells (ATCC, USA; CRL-1573^{™}) which is a human embryonic kidney cell line by using lipofectamine. After culturing the transfected cells for 3 days, the IgG expressed and produced in the supernatant was isolated and purified by using a protein G affinity column. The purified proteins were analyzed by using SDS-PAGE and were found to have a purity of at least 95 %.

### Example 3. Selection of antibodies showing high binding affinity to cancer cells

To select antibodies showing high binding strength to cancer cells among the antibodies selected in Example 2, flow cytometry was performed as follows.

Cancer cells (patient-derived cells, PDC) obtained from survivors of 5 or more years after triple-negative breast cancer surgery were treated with trypsin, and resuspended in a FACS buffer solution (3 % FBS in PBS) to a final concentration of 1×10⁶ cells/ml. 2 µg of each of the seven antibodies selected in Example 2 was cultured with PDC for 2 hours, and then allowed to bind to Fluorescein (FITC)-conjugated AffiniPure Goat-Anti Human IgG (109-095-098, Jackson ImmunoResearch) for 1 hour. After the binding, the cells were filtered by using a 100 µm strainer. The filtered cells were evaluated by using a BD Accuri^{™} C6 flow cytometer and a BD Accuri^{™} C6 Plus flow cytometer, and analyzed by using a BD Accuri C6 software and a BD Accuri C6 Plus software.

As a result, the S3-5 and S4-2 antibodies were commonly found to have excellent binding strength to the cancer cells (FIG. 1), and then selected.

### Example 4. Confirmation of anticancer efficacy of selected antibodies

### Example 4.1 In vitro cell proliferation assay

To confirm effects of the S3-5 and S4-2 antibodies selected in Example 3 on the growth of cancer cells, the following experiments were performed. PDCs which are cancer cells obtained from survivors of 5 or more years after triple-negative breast cancer surgery were inoculated onto a 96-well plate at a density of 3,000 cells/well to 10,000 cells/well in a 50 µl culture medium. After the inoculation, cancer cells were treated with the five antibodies (S3-2, S3-5, S4-2, S4-4, S4-9) identified in Example 2 at concentrations of 0.2 µg/ml, 1 µg/ml, and 5 µg/ml.

To monitor the cell proliferation, 1/10 volume of Quanti-Max^{™} reagent was added directly to a untreated control and antibody-treated cells 24 and 48 hours after the antibody treatment. After incubating the cancer cells for an additional 3 hours, the viability of the cells was measured by fluorescence measurement according to microplate fluorescence spectrophotometry.

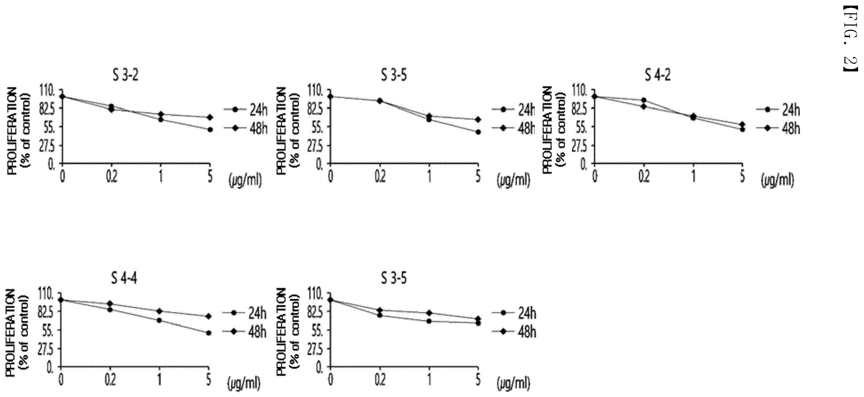

As a result, it was confirmed that the S3-5 and S4-2 antibodies inhibited the growth of the cancer cells in a concentration-dependent manner compared to other antibodies (FIG. 2).

### Example 4.2 In vitro cell migration assay

To confirm effects of the S3-5 and S4-2 antibodies selected in Example 3 on the migration of cancer cells, the following experiments were performed. The migration ability of cells was analyzed by using a transwell chamber with a 6.5 mm-diameter polycarbonate filter (pore size of 8.0 µm). MDA-MB-468 cells which are the breast cancer cell line were treated with trypsin and resuspended in a serum-free medium to a final concentration of 2 to 4 × 10⁴ cells/ml.

The cell suspension was spread into an upper chamber, and 5 µg each of the five antibodies (S3-2, S3-5, S4-2, S4-4, and S4-9) identified in Example 2 was treated. 10 % FBS was used in a lower chamber, and the chamber was cultured at 37°C for 24 hours. After completion of the culture, the cells were fixed and stained with hematoxylin and eosin. Non-migrating cells in the upper chamber of the filter were removed by wiping with a cotton swab, and the migration ability was measured by photographing and counting cells that migrated to the lower part of the filter by using an optical microscope.

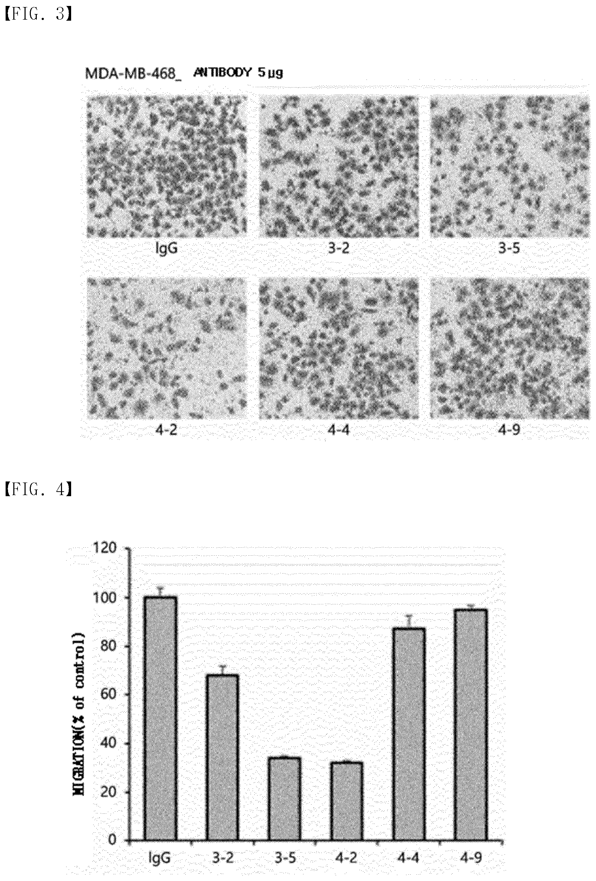

As a result, it was confirmed that the S3-5 and S4-2 antibodies inhibited the migration of the cancer cells compared to other antibodies (FIGS. 3 and 4).

### Example 4.3 In vivo experiments in animal model with lung metastasis

To confirm effects of the S3-5 and S4-2 antibodies selected in Example 3 on the metastasis of cancer cells in an animal model of breast cancer with lung metastasis, the following experiments were performed. Here, an immunodeficient NSG mouse (JA Bio, Korea) injected with MDA-MB-468 cells which are a human breast cancer cell line was used as an animal model of breast cancer with lung metastasis. In detail, 5 × 10⁵ MDA-MB-468 cells were suspended in 150 µl of PBS and injected into the tail vein of the NSG mouse. Three days after the injection of the cancer cells, IgG (2.5mg/kg) in a control group and S3-5 (2.5 mg/kg) antibody and S4-2 (2.5 mg/kg) antibody in experimental groups were each administered intravenously twice a week for 3 weeks.

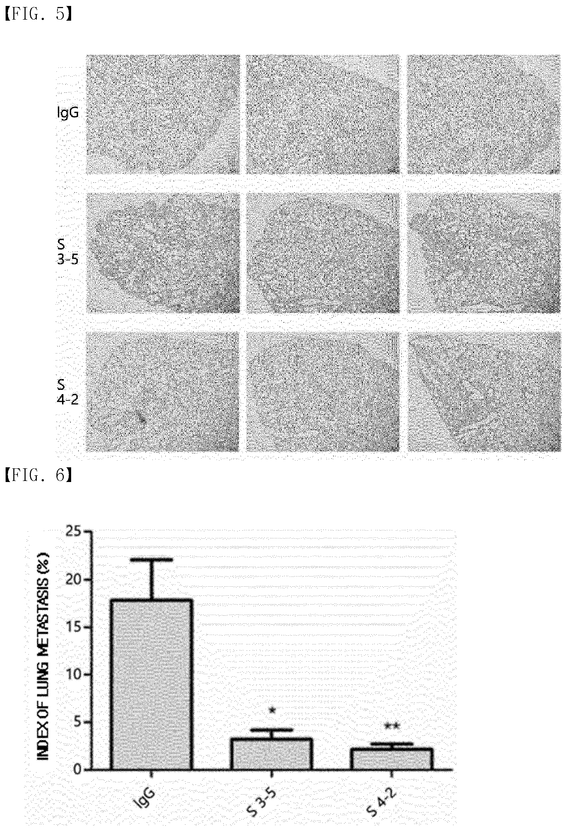

After three weeks, the mouse was euthanized, the lungs were extracted and fixed with formalin, and tissue slide sections were obtained through a paraffin embedding process. These tissue slides were stained with H&E and found that the percentage of lung metastasis was significantly reduced in the mouse model having breast cancer with lung metastasis treated with the S3-5 and S4-2 antibodies, compared to the control group (FIG. 5). To quantify this, the area of a part where the cancer cells have metastasized is calculated by using Image J and divided by the total area to obtain the metastatic index. As shown in FIG. 6, a significant decrease in the metastatic index was confirmed compared to the control group, when the S3-5 and S4-2 antibodies were administered as shown in FIG. 6.

### Example 4.4 Antibody-dependent cell cytotoxicity (ADCC)

In a 96-well plate, MDA-MB-468 cells, which are the breast cancer cell line, and PDCs, which are obtained from survivors of 5 or more years after triple-negative breast cancer surgery, were each seeded at a density of 1 × 10⁴/100 µℓ/well, and the cells were cultured at 37°C for 24 to 48 hours to prepare cancer cells that are target cells.

8 ml of peripheral blood was collected from breast cancer patients and normal controls, and whole blood was diluted with citrate-dextrose solution (ACD solution, Sigma; C3821) or PBS at a 1:1 ratio. In a 15 ml conical tube, 3 ml of Ficoll-Paque (Miltenyl Biotec) was added, and 10 ml of diluted blood was carefully added thereto. Then, centrifugation was performed thereon at 400 g (or 2,000 rpm to 2,500 rpm) by using a swinging bucket rotor without a brake for 30 minutes to 40 minutes (or 20 minutes to 30 minutes) at room temperature. The upper layer was removed by using a pipette, leaving a buffy coat (containing PBMC) at the interface. After carefully transferring the PBMC layer to a new 15 ml conical tube, the tube was filled with PBS and centrifuged at 200 g (or 1,500 rpm to 1,800 rpm) for 10 minutes to 15 minutes (or 5 minutes). The supernatant was discarded, and the pellets were resuspended in PBS and recentrifuged at 1,500 rpm for 5 minutes. PBMCs were prepared by resuspending the pellets in PBS and counting the cells.

A carboxyfluorescein succinimidyl ester (CFSE, Invitrogen; final concentration of 5 uM, 100 µl/well) solution was added to the PBMCs at 37°C for 20 minutes to stain the plated breast cancer cells. After removing CFSE that did not bind to the cancer cells, the stained cancer cells were washed twice for 5 minutes each using DPBS containing 10 % FBS.

Each of the S3-5 and S4-2 antibodies was added to wells plated with the stained cancer cells, at a concentration of 1 µg/100 µl/well and cultured at 37°C for 1 hour. As a control, Fc gamma blocking antibody was added at the same concentration 1 hour before the addition of the S3-5 and S4-2 antibodies. Next, PBMC cell suspension was added to the wells at a density of 2.5 × 105/100 µl/well (cancer cell: PBMC at a ratio of 1:25) and cultured at 37 °C overnight (for 16 hours). After the completion of the culture, floating cells and attached cells were obtained (by adding trypsin/EDTA), and the cell suspension was transferred to a new E. tube. Then, centrifugation was performed thereon at 3,000 rpm for 5 minutes at 4 °C, and the cell pellets were resuspended and stained with propidium bromide (BD, #. 51-66211E). The single cell suspension was analyzed for CSFE and PI double positive cells by using a flow cytometer (Accuri C6, BD).

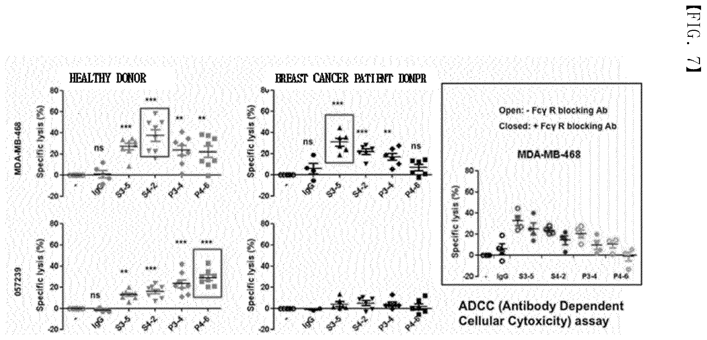

As a result, it was confirmed that the S3-5 and S4-2 antibodies promoted apoptosis of the cancer cells by activation of immune cells (FIG. 7).

### Example 5. Identification of antigens for S3-5 and S4-2 antibodies

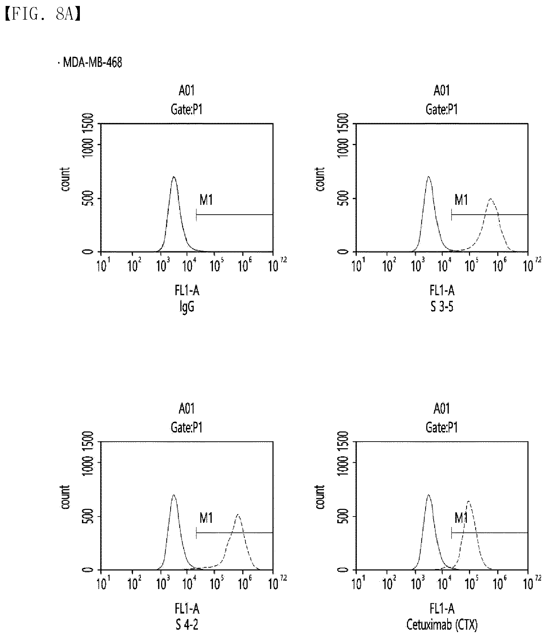

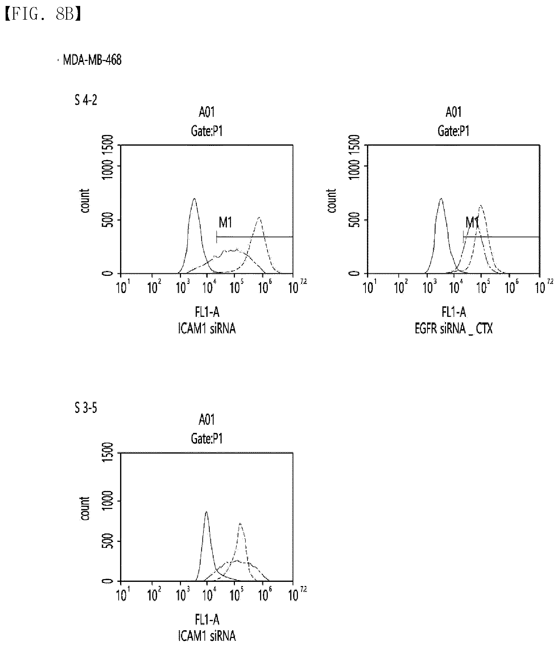

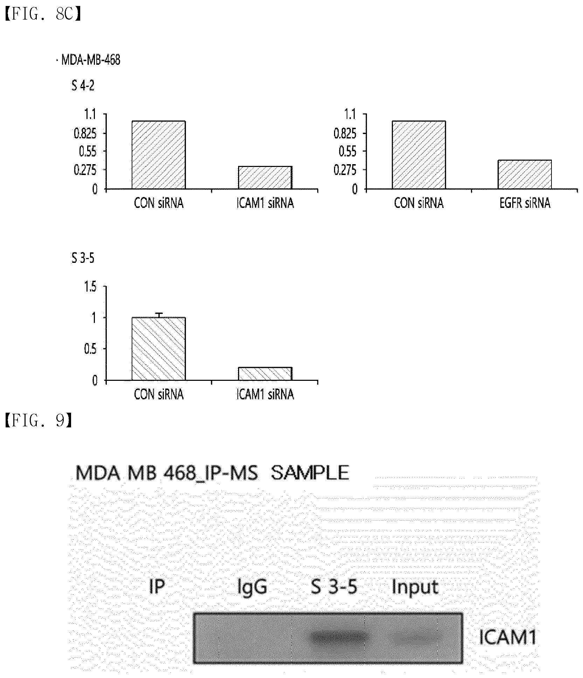

To identify antigens binding to the S3-5 and S4-2 antibodies selected in Example 3, immunoprecipitation-mass spectrometry was performed as follows. First, the S3-5 and S4-2 antibodies were mixed with lysate of MDA-MB-468 cells which are the breast cancer cell line, and captured with protein G agarose conjugated beads (immunoprecipitation reaction). Through this, mass spectrometry was performed by using LC-MS/MS equipment to secure proteins binding to the antibodies and confirm identification of the proteins. To determine which genes were essential for the binding of the S3-5 and S4-2 antibodies among the candidate antigen genes detected by mass spectrometry, specific genes were knockdown by using siRNA, and FACS was utilized to test whether there was a change in the antibody binding. As a positive control, Cetuximab which is a well-known antibody to EGFR was used to confirm that siEGFR treatment reduced the binding of Cetuximab. Similarly, the binding of the S3-5 and S4-2 antibodies was reduced when the expression of the ICAM1 gene was inhibited by treatment with ICAM1 siRNA (FIG. 8). Through this, it was confirmed that the antigens of the S3-5 and S4-2 antibodies were ICAM1.

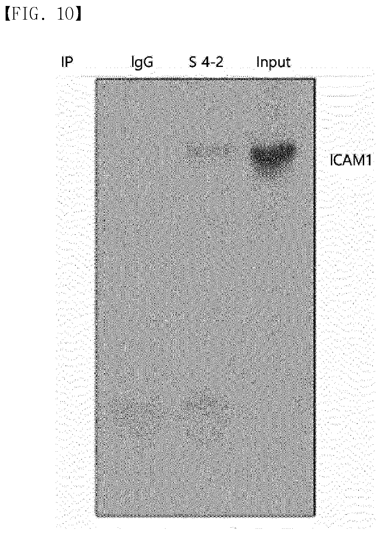

In addition, the presence of ICAM1 in the reaction product of the immunoprecipitation of the S3-5 and S4-2 antibodies was confirmed through western blot, confirming that the antigens of the S3-5 and S4-2 antibodies were ICAM1 (FIGS. 9 and 10).

## Claims

1. An anti-ICAM1 antibody or an antigen-binding fragment thereof, comprising: a heavy chain variable region comprising a heavy chain CDR1 having an amino acid sequence of SEQ ID NO: 1 or 10, a heavy chain CDR2 having an amino acid sequence of SEQ ID NO: 2 or 11, and a heavy chain CDR3 having an amino acid sequence of SEQ ID NO: 3 or 12; and a light chain variable region comprising a light chain CDR1 having an amino acid sequence of SEQ ID NO: 5 or 14, a light chain CDR2 having an amino acid sequence of SEQ ID NO: 6 or 15, and a light chain CDR3 having an amino acid sequence of SEQ ID NO: 7 or 16.

2. The antigen-ICAM1 antibody or the antigen-binding fragment thereof of claim 1, comprising the heavy chain variable region and the light chain variable region of one of the following combinations:
a) the heavy chain variable region comprising the heavy chain CDR1 having the amino acid sequence of SEQ ID NO: 1, the heavy chain CDR2 having the amino acid sequence of SEQ ID NO: 2, and the heavy chain CDR3 having the amino acid sequence of SEQ ID NO: 3; and the light chain variable region comprising the light chain CDR1 having the amino acid sequence of SEQ ID NO: 5, the light chain CDR2 having the amino acid sequence of SEQ ID NO: 6, and the light chain CDR3 having the amino acid sequence of SEQ ID NO: 7; and
b) the heavy chain variable region comprising the heavy chain CDR1 having the amino acid sequence of SEQ ID NO: 10, the heavy chain CDR2 having the amino acid sequence of SEQ ID NO: 11, and the heavy chain CDR3 having the amino acid sequence of SEQ ID NO: 12; and the light chain variable region comprising the light chain CDR1 having the amino acid sequence of SEQ ID NO: 14, the light chain CDR2 having the amino acid sequence of SEQ ID NO: 15, and the light chain CDR3 having the amino acid sequence of SEQ ID NO: 16.

3. The antigen-ICAM1 antibody or the antigen-binding fragment thereof of claim 1, wherein
the heavy chain variable region has an amino acid sequence of SEQ ID NO: 4 or 13, and the light chain variable region has an amino acid sequence of SEQ ID NO: 8 or 17.

4. The anti-ICAM1 antibody or the antigen-binding fragment thereof of claim 3, comprising the heavy chain variable region and the light chain variable region of one of the following combinations:
a) the heavy chain variable region having the amino acid sequence of SEQ ID NO: 4, and the light chain variable region having the amino acid sequence of SEQ ID NO: 8; and
b) the heavy chain variable region having the amino acid sequence of SEQ ID NO: 13, and the light chain variable region having the amino acid sequence of SEQ ID NO: 17.

5. A polynucleotide encoding the antibody or the antigen-binding fragment thereof of any one of claims 1 to 4.

6. A recombinant vector comprising the polynucleotide of claim 5.

7. A host cell transformed by introducing the recombinant vector of claim 6.

8. A method of preparing an antibody or an antigen-binding fragment thereof, the method comprising:
culturing the host cell of claim 7 that is transformed; and
obtaining an antibody or an antigen-binding fragment thereof, from the cultured host cell.

9. A pharmaceutical composition for treating or preventing cancer, comprising the antibody or the antigen-binding fragment thereof of any one of claims 1 to 4.

10. The pharmaceutical composition of claim 9, wherein the cancer is one selected from the group consisting of breast cancer, stomach cancer, liver cancer, lung cancer, colon cancer, colorectal cancer, prostate cancer, ovarian cancer, pancreatic cancer, cervical cancer, thyroid cancer, laryngeal cancer, renal cancer, skin cancer, acute myeloid leukemia, brain tumor, neuroblastoma, retinoblastoma, head and neck cancer, salivary gland cancer, and lymphoma.

11. Use of an anti-ICAM1 antibody or an antigen-binding fragment thereof for preventing or treating cancer, the anti-ICAM1 antibody or the antigen-binding fragment thereof comprising: a heavy chain variable region comprising a heavy chain CDR1 having an amino acid sequence of SEQ ID NO: 1 or 10, a heavy chain CDR2 having an amino acid sequence of SEQ ID NO: 2 or 11, and a heavy chain CDR3 having an amino acid sequence of SEQ ID NO: 3 or 12; and a light chain variable region comprising a light chain CDR1 having an amino acid sequence of SEQ ID NO: 5 or 14, a light chain CDR2 having an amino acid sequence of SEQ ID NO: 6 or 15, and a light chain CDR3 having an amino acid sequence of SEQ ID NO: 7 or 16.

12. A method of preventing or treating cancer, the method comprising administering to a subject a composition comprising an anti-ICAM1 antibody or an antigen-binding fragment thereof as an active ingredient, the anti-ICAM1 antibody or the antigen-binding fragment thereof comprising: a heavy chain variable region comprising a heavy chain CDR1 having an amino acid sequence of SEQ ID NO: 1 or 10, a heavy chain CDR2 having an amino acid sequence of SEQ ID NO: 2 or 11, and a heavy chain CDR3 having an amino acid sequence of SEQ ID NO: 3 or 12; and a light chain variable region comprising a light chain CDR1 having an amino acid sequence of SEQ ID NO: 5 or 14, a light chain CDR2 having an amino acid sequence of SEQ ID NO: 6 or 15, and a light chain CDR3 having an amino acid sequence of SEQ ID NO: 7 or 16.
